(19) Europäisches Patentamt
European Patent Office
Office européen des brevets

(11) Veröffentlichungsnummer: **0 079 022**
**B1**

(12) # EUROPÄISCHE PATENTSCHRIFT

(45) Veröffentlichungstag der Patentschrift: **12.03.86**

(51) Int. Cl.⁴: **C 07 D 209/52**, C 07 C 101/28, C 07 C 101/34, A 61 K 31/40

(21) Anmeldenummer: **82110070.8**

(22) Anmeldetag: **02.11.82**

(54) **Derivate der cis, endo-2-Azabicyclo-(3.3.0)-octan-3-carbonsäure, Verfahren zu ihrer Herstellung, diese enthaltende Mittel und deren Verwendung.**

(30) Priorität: **05.11.81 DE 3143946**
**17.07.82 DE 3226768**

(43) Veröffentlichungstag der Anmeldung: **18.05.83 Patentblatt 83/20**

(45) Bekanntmachung des Hinweises auf die Patenterteilung: **12.03.86 Patentblatt 86/11**

(84) Benannte Vertragsstaaten: **AT BE CH DE FR GB IT LI LU NL SE**

(56) Entgegenhaltungen:
**EP - A - 0 037 231**
**DE - A - 1 955 375**

**CHEMICAL ABSTRACTS, Band 69, Nr. 9, 26. August 1968, Seite 3337, Zusammenfassung Nr. 35847h, COLUMBUS OHIO US**
**CHEMICAL ABSTRACTS, Band 89, Nr. 25, 18. Dezember 1978, Seite 630, Zusammenfassung Nr. 215732p, COLUMBUS OHIO (US)**
**CHEMICAL ABSTRACTS, Band 88, Nr. 5, 30. Januar 1978, Seite 470, Zusammenfassung Nr. 37442p, COLUMBUS OHIO (US)**
**CHEMICAL ABSTRACTS, Band 89, Nr. 15, 9. Oktober 1978, Seite 595, Zusammenfassung Nr. 129529w, COLUMBUS OHIO (US)**

(73) Patentinhaber: **HOECHST AKTIENGESELLSCHAFT, Postfach 80 03 20, D-6230 Frankfurt am Main 80 (DE)**

(72) Erfinder: **Teetz, Volker, Dr., An der Tann 20, D-6238 Hofheim am Taunus (DE)**
Erfinder: **Geiger, Rolf, Prof. Dr., Heinrich-Bleicher-Strasse 33, D-6000 Frankfurt am Main 50 (DE)**
Erfinder: **Urbach, Hansjörg, Dr., Le Lavandou Strasse 41, D-6242 Kronberg/Taunus (DE)**
Erfinder: **Becker, Reinhard, Dr., Adelheidstrasse 101, D-6200 Wiesbaden (DE)**
Erfinder: **Schölkens, Bernward, Dr., Am Fliedergarten 1, D-6233 Kelkheim (Taunus) (DE)**

**EP 0 079 022 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus der EP-A2-37231 sind substituierte Acylderivate der Octahydro-1H-indol-2-carbonsäure und deren Verwendung als Hemmer des Angiotensin-Converting-Enzyms (ACE) bekannt.

Es wurde nun überraschenderweise gefunden, dass Derivate der cis, endo-2-Azabicyclo[3.3.0]-octan-3-carbonsäure starke ACE-Hemmer sind.

Die Erfindung betrifft daher Verbindungen der Formel I'

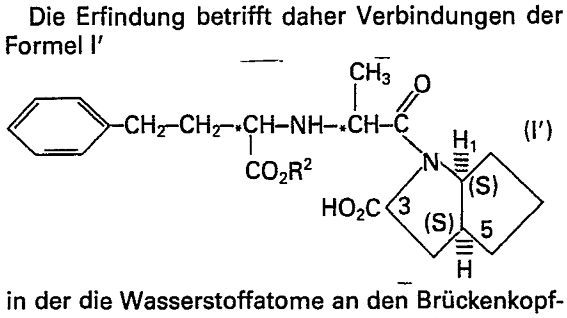

(I')

in der die Wasserstoffatome an den Brückenkopf-C-Atomen 1 und 5 zueinander cis-konfiguriert sind, die Carboxygruppen am C-Atom 3 endoständig zum bicyclischen Ringsystem orientiert (d. h. dem Cyclopentanring zugewandt) ist, und die Chiralitätszentren an den mit einem Stern (*) markierten beiden C-Atomen der Kette und am C-Atom 3 des Bicyclus in der S-Konfiguration vorliegen und in der $R^2$ = Wasserstoff, Methyl, Ethyl oder Benzyl bedeutet sowie deren physiologisch unbedenklichen Salze.

Bevorzugte Verbindungen sind:
N-(1-S-Carbethoxy-3-phenyl-propyl)-S-alanyl-cis, endo-2-azabicyclo-[3.3.0]-octan-3-S-carbonsäure und N-(1-S-Carboxy-3-phenyl-propyl)-S-alanylcis, endo-2-azabicyclo-[3.3.0]-octan-3-S-carbonsäure sowie jeweils deren physiologisch unbedenklichen Salze.

Als Salze kommen insbesondere in Frage die Hydrochloride, Maleinate, Tartrate bzw. die Alkali-, Ca-, Mg- und Zn-Salze.

Die Erfindung betrifft ferner ein Verfahren zur Herstellung der Verbindungen der Formel I', das dadurch gekennzeichnet ist, dass man eine Verbindung der Formel II'

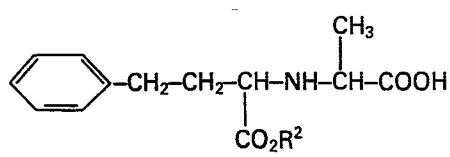

(II')

in der $R^2$ die oben definierte Bedeutung mit Ausnahme von $R^2$ = Wasserstoff hat, mit einer Verbindung der Formel III

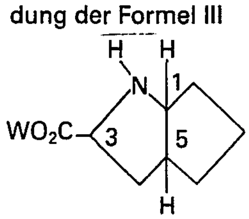

(III)

in der die Wasserstoffatome an den Brückenkopf-C-Atomen 1 und 5 zueinander cis-konfiguriert sind und die $CO_2W$-Gruppe am C-Atom 3 endoständig zum bicyclischen Ringsystem orientiert (d. h. dem Cyclopentanring zugewandt) ist und in der W eine Carboxy veresternde Gruppe, vorzugsweise tert. Butyl oder Benzyl bedeutet, nach bekannten Amidbildungsmethoden der Peptidchemie umsetzt und anschliessend das Produkt hydriert oder mit einer Säure oder einer Base behandelt und die erhaltene Verbindung gegebenenfalls in ihr physiologisch verträgliches Salz überführt.

Verbindungen der Formel II' mit $R^2 = CH_3$ oder $C_2H_5$ sind beispielsweise aus der EP-A2-37231 bekannt und auf verschiedenen Wegen zugänglich. Die Benzylester ($R^2$ = Benzyl) können analog hergestellt werden.

Es wurde gefunden, dass die Mannich-Reaktion von Acetophenon der Formel IVa mit Glyoxylsäureestern und Alaninestern zu Verbindungen der Formel IV führt. In Formel IV bedeutet W' einen hydrogenolytisch, basisch oder sauer abspaltbaren Rest, bevorzugt Benzyl oder tert. Butyl und $R^2$ hat die vorstehend definierten Bedeutungen. Im Falle des Benzylesters (W' = Benzyl) darf jedoch $R^2$ nicht Benzyl sein. Bei der Hydrogenolyse dieser Verbindungen mit Pd entstehen Verbindungen der Formel II'.

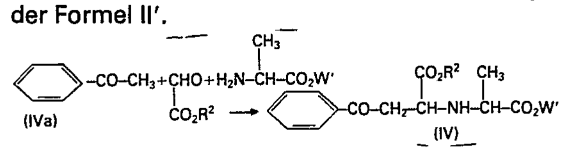

(IVa) (IV)

Verbindungen der Formel IV können ebenfalls durch Michael-Addition entsprechender Keto-Acrylsäureester mit Alaninestern in hohen Ausbeuten gewonnen werden. Die Esterspaltung führt zu denselben Produkten wie die Mannich-Reaktion.

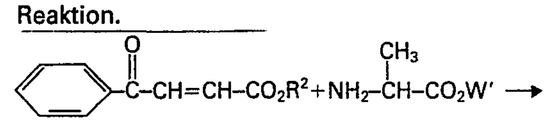

IV

Die Diastereomeren mit der bevorzugten S,S-Konfiguration entstehen bei Einsatz von L-Alaninestern in überwiegender Menge und können durch Kristallisation oder chromatographische Trennung der Ester von II' an Kieselgel gewonnen werden.

Die Erfindung betrifft weiterhin Verbindungen der Formel III

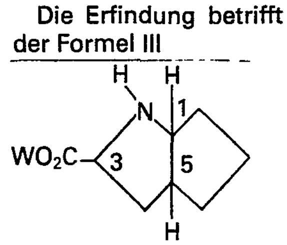

(III)

in der die Wasserstoffatome an den Brückenkopf-C-Atomen 1 und 5 zueinander cis-konfiguriert sind und die $CO_2W$-Gruppe am C-Atom 3 endoständig zum bicyclischen Ringsystem orientiert (d. h. dem Cyclopentanring zugewandt) ist und in der W Wasserstoff oder eine Carboxy veresternde Gruppe, die durch Hydrieren oder durch Behandeln mit einer Säure oder Base abspaltbar ist, vorzugsweise tert. Butyl oder Benzyl bedeutet sowie deren Salze sowie ein Verfahren zur Herstellung dieser Verbindungen, das dadurch gekennzeichnet ist, dass man jene Verbindung, die erhalten wird, indem man eine Verbindung der Formel X

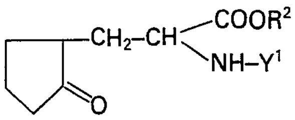

(X)

in welcher $R^2$ eine sauer abspaltbare Estergruppe und $Y^1$ einen Acylrest bedeuten, unter sauren Bedingungen cyclisiert, durch katalytische Hydrierung in Gegenwart von Edelmetallkatalysatoren oder durch Reduktion mit komplexen Hydriden in Verbindungen der Formel III, in welcher W für Wasserstoff steht, überführt, diese gegebenenfalls zu Verbindungen der Formel III, in welcher W die Bedeutungen wie oben mit der Ausnahme von Wasserstoff hat, verestert und die erhaltenen Verbindungen gegebenenfalls in ihre Salze überführt.

Die Ausgangsverbindungen des Herstellungsverfahrens für Verbindungen der Formel III werden erhalten durch Umsetzung eines Enamins der Formel VI, in welcher $X^1$ für Dialkylamine mit 2 bis 10 C-Atomen oder für einen Rest der Formel VII, worin m und o eine ganze Zahl von 1 bis 3, $(m + o) \geq 3$ und A $CH_2$, NH, O oder S bedeuten, steht,

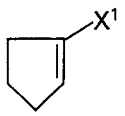

(VI)

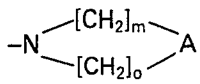

(VII)

mit einem N-acylierten β-Halogen-α-amino-carbonsäureester der Formel VIII, in welcher $X_2$ für eine nucleofuge Gruppe, $Y^1$ für Alkanoyl mit 1 bis 5 C-Atomen, Aroyl mit bis 9 C-Atomen oder andere, in der Peptidchemie übliche, sauer abspaltbare Schutzgruppen und $R^2$ für Alkyl mit 1 bis 5 C-Atomen oder Aralkyl mit 7 bis 9 C-Atomen steht

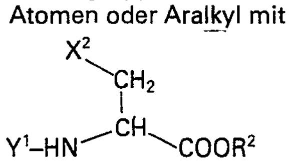

(VIII)

oder mit einem Acrylsäureester der Formel IX, in welcher $Y^1$ und $R^2$ vorstehende Bedeutung haben

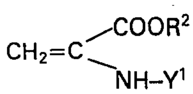

(IX)

zu einer Verbindung der Formel X, in welcher $R^2$ und $Y^1$ vorstehende Bedeutung haben,

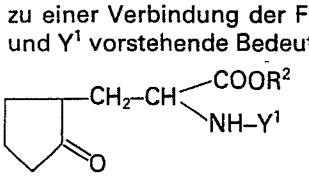

(X)

und Cyclisierung dieser Verbindung mit Hilfe starker Säuren unter Acylamid- und Esterspaltung.

Die bicyclischen Verbindungen der Formel III besitzen die cis,endo-Konfiguration, d.h. die $-CO_2W$-Gruppe ist dem Cyclopentanring zugewandt. Sie liegen vorzugsweise in der all-S-Konfiguration vor. Auch alle weiteren in der vorliegenden Erfindung aufgeführten 2-Azabicyclo-[3.3.0]-octan-3-carbonsäure-Derivate liegen in der cis,endo-Kondifuration vor.

Bevorzugte Enamine sind beispielsweise Pyrrolidinocyclopenten und Morpholinocyclopenten. Die Cyclisierung der Alkylierungsprodukte der Formel X erfolgt vorzugsweise mit wässriger Chlorwasserstoffsäure. Die Verbindungen der Formel III (mit W = H) können nach den bei Aminosäuren üblichen Methoden [s. z. B. Houben-Weyl, Methoden der organischen Chemie, Bd. VIII (1952)] verestert werden, z. B. mit Thionylchlorid/Benzylalkohol oder Isobutylen/Schwefelsäure. Sie führt nach entsprechender Aurarbeitung zu Verbindungen der Formel III in Form der freien Base oder eines Salzes.

Die neuen Verbindungen der Formel I′ besitzen eine langdauernde, intensive blutdrucksenkende Wirkung. Sie sind starke Hemmer des Angiotensin-Converting-Enzyms (ACE-Hemmer) und können zur Bekämpfung des Bluthochdrucks verschiedener Genese eingesetzt werden. Auch ihre Kombination mit anderen blutdrucksenkenden gefässerweiternden oder diuretisch wirksamen Verbindungen sind möglich. Typische Vertreter dieser Wirkklassen sind z. B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1–100 mg je Einzeldosis. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemässen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragées, Steckkapseln, wässrige alkoholische oder ölige Suspensionen oder wässrige alkoholische oder ölige Lösungen. Als inerte Träger können z. B. Gummi arabicum, Magnesiumcarbonat, Kaliumphosphat, Milchzukker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel für die neuen aktiven Verbindungen und die entsprechenden physiologisch verträglichen Salze kommen z. B. in Frage: Wasser, physiologische Kochsalzlösungen oder Alkohole, z. B. Ethanol, Propandiol oder Glycerin, daneben auch Zuckerlösungen wie Glukose- oder

Mannitlösungen, oder auch eine Mischung aus den verschiedenen genannten Lösungsmitteln.

Die ausserordentlich starke Wirksamkeit der Verbindungen gemäss Formel I' – auch bei oraler Gabe – wird durch nachfolgende pharmakologische Daten belegt.

Intravenöse Applikation an der narkotisierten Ratte, 50% Hemmung der durch 310 ng Angiotensin I ausgelösten Pressorreaktion 30 min nach Applikation in der Dosis ... $ED_{50}$

| $R^2$ | $ED_{50}$ (μg/kg) |
|---|---|
| $C_2H_5$ | 8,3 |
| H | 2,7 |

| $R^2$ | $ED_{50}$ (μg/kg) |
|---|---|
| $C_2H_5$ | 50 |
| H | 600 |

Bei oraler Gabe an der wachen Ratte zeigt sich in der Dosierung von 1 mg/kg bei z. B. der Verbindung der Formel I' mit $R^2$ = Ethyl eine über 6 h anhaltende, über 90%ige Hemmung der durch i.V. appliziertes Angiotensin I ausgelösten Pressorreaktion.

Die nachfolgenden Beispiele sollen die erfindungsgemässen Verfahrensweisen erläutern, ohne die Erfindung auf die hier stellvertretend genannten Substanzen zu beschränken.

Beispiel I

N-(1-S-Carboethoxy-3-phenyl-propyl)-S-alanyl-2-cis,endo-azabicyclo-[3.3.0]-octan-3-S-carbonsäure

(1) 2-Acetylamino-3-(2-oxo-cyclopentyl)-propionsäuremethylester

269 g 3-Chlor-2-acetyl-amino-propionsäuremethylester und 257 g Cyclopentenopyrrolidin werden in 1,5 l DMF 24 h bei Raumtemperatur gehalten. Man engt im Vakuum ein, nimmt den Rückstand in wenig Wasser auf, stellt mit konzentrierter Salzsäure auf pH 2 und extrahiert 2 mal mit je 4 l Essigester. Beim Einengen der organischen Phase hinterbleibt ein hellgelbes Öl.

Ausbeute: 290 g.

NMR: 2,02 (s, 3H); 3,74 (s, 3H); 4,4–4,8 (m, 1H) ($CDCl_3$)

Analyse:
ber.: C 58,1 H 7,54 N 6,16
gef.: C 58,5 H 7,2 N 6,5

(2) cis,endo-2-Azabicyclo-[3.3.0]-octan-3-carbonsäurehydrochlorid

270 g des unter (1) hergestellten Acetylamino-Derivates werden in 1,5 l 2n Salzsäure 45 min am Rückfluss gekocht. Man engt im Vakuum ein, nimmt den Rückstand in Eisessig auf, versetzt mit 5 g Pt/C (10% Pt) und hydriert bei 5 bar. Nach Filtration wird eingeengt und der Rückstand aus Chloroform/Diisopropylether kristallisiert.

Schmelzpunkt: 205–209°C,
Ausbeute: 150 g

(3) cis,endo-2-Azabicyclo-[3.3.0]-octan-3-carbonsäurebenzylester-hydrochlorid

40 g der unter (2) hergestellten Carbonsäure werden in eine eiskalte Mischung aus 390 g Benzylalkohol und 65 g Thionylchlorid gegeben und 24 h bei Raumtemperatur belassen. Nach Einengen im Vakuum kristallisieren 47 g des Benzylesters aus Chloroform/Isopropanol.

Schmelzpunkt: 175°C (Hydrochlorid)

(4) N-(1-S-Carbethoxy-3-phenyl-propyl)-S-alanyl-cis,endo-2-azabicyclo-[3.3.0]-octan-3-S-carbonsäurebenzylester

14 g des nach (3) hergestellten Benzylesters werden mit 6,7 g HOBt, 13,8 g N-(1-S-Carbethoxy-3-phenyl-propyl)-S-alanin, und 10,2 g Dicyclohexylcarbodiimid in 200 ml Dimethylformamid zur Reaktion gebracht. Nach 3 h Rühren bei Raumtemperatur saugt man von ausgefallenem Dicyclohexylharnstoff ab, engt ein, nimmt in 1 l Essigester auf und schüttelt mit 3×500 ml 5%iger $NaHCO_3$-Lösung aus. Die organische Phase wird eingeengt und mit Essigester/Petrolether im Verhältnis 2:1 über eine Säule aus 1 kg Kieselgel chromatographiert. Das zuerst eluierte Isomere stellt die S,S,S-Verbindung dar, ein späteres Eluat liefert nach dem Einengen die S,S,R-Verbindung.

Es werden jeweils 8,0 g Produkt als Öl erhalten.

NMR: der S,S,S-Verbindung: Charakteristische Signale:
1,20 (d, 3H), 1,27 (t, 2H), 4,17 (q, 3H), 5,13 (s, 2H), 7,18 (s, 5H), 7,32 (s, 5H) ($CDCl_3$)

Analyse $C_{30}H_{38}N_2O_5$
ber.: C 71,1 H 7,56 N 5,53
gef.: C 70,8 H 7,8 N 5,7

(5) N-(1-S-Carboethoxy-3-phenyl-propyl)-S-alanyl-cis,endo-2-azabicyclo[3.3.0]-octan-3-S-carbonsäure

8,0 g des S,S,S-Benzylesters aus (4) werden in 100 ml Ethanol gelöst und unter Zusatz von 0,5 g 10% Pd/C bei Normaldruck hydrogenolisch entbenzyliert. Diese Reaktion kann auch unter Druck bei gleichzeitiger Verkürzung der Reaktionszeit vorgenommen werden. Nach Aufnahme der berechneten Menge Wasserstoff wird vom Katalysator abfiltriert und im Vakuum eingeengt. Das Zwitterion kristallisiert in fast quantitativer Ausbeute aus Ether:

Schmelzpunkt: 110–112°C (Zers.)

Durch Zusatz einer äquivalenten Menge Salzsäure kann ein Hydrochlorid (ab 120°C Zersetzung) oder durch Zugabe von wässrigen Zinksalzen zu einer konzentrierten methanolischen Lösung der Titelverbindung ein thermisch besonders stabiles Zink-Komplexsalz (Zersetzung über 160°C) erhalten werden.

Analyse $C_{23}H_{32}N_2O_5$:
ber.: C 66,3 H 7,7 N 6,73
gef.: C 66,1 H 7,8 N 6,6

Die erhaltenen NMR- und Massenspektren sind im Einklang mit der angegebenen Struktur.

$[\alpha]_D = +15{,}6°$ (c = 1, Methanol).

Beispiel II

(1) cis,endo-2-Azabicyclo-[3.3.0]-octan-3-carbonsäure-tert.butylester

25 g Azabicyclo-[3.3.0]-octancarbonsäure-hydrochlorid aus Beispiel I (2) werden in 250 ml Dioxan mit 250 ml Isobutylen und 25 ml konzentrierter Schwefelsäure zur Reaktion gebracht. Nach 14 h bei Raumtemperatur wird mit Natronlauge alkalisch gestellt, im Vakuum eingeengt, mit 100 ml Wasser versetzt und der Ester ausgeethert. Nach Abdampfen des Ethers erhält man 15 g farbloses Öl.

Analyse $C_{12}H_{21}NO_2$:
ber.: C 68,2 H 10,2 N 6,63
gef.: C 67,9 H 10,1 N 6,3

Beispiel III

Allgemeine Methode: Esterverseifung zur Darstellung von Verbindungen der Formel I' mit $R^2 = H$.

10 g des entsprechenden Ethyl- oder Benzylesters der Formel I' werden in 200 ml Dimethoxyethan gelöst. Man fügt einen Tropfen einer verdünnten Indikatorlösung, z. B. Bromthymolblau, zu und fügt unter starkem Rühren im Verlauf von 5 min eine äquivalente Menge 4n KOH (wässrig) hinzu, so dass der Indikator bei Beendigung der Reaktion einen pH-Wert von 9–10 anzeigt. Sodann stellt man mit Salzsäure auf pH 4, engt im Vakuum zur Trockene ein, nimmt in 250 ml Essigester auf und filtriert. Beim Einengen des Essigesters fallen die Dicarbonsäuren als feste, kristalline oder amorphe Verbindungen an.

Die Ausbeuten liegen zwischen 80 und 95%.

Beispiel IIIa

N-(1-S-Carboxy-3-phenyl-propyl)-S-alanyl-cis, endo-2-azabicyclo-[3.3.0]-octan-3-S-carbonsäure

1 g N-(1-S-Carbethoxy-3-phenyl-propyl)-S-alanyl-2-azabicyclo-[3.3.0]-octan-3-S-carbonsäure aus Beispiel I (5) wird wie unter Beispiel III beschrieben verseift (1 h) und aufgearbeitet.

Ausbeute: 0,85 g
m/e: 388

Beispiel IV

N-(1-S-Carbethoxy-3-oxo-3-phenyl-propyl)-S-alanin-benzylester

65,7 g 3-Phenyl-3-oxo-1-carbonsäureethylester (Benzoylacrylsäureethylester) werden in 225 ml Ethanol gelöst und dazu 1 ml Triethylamin gegeben. Zu dieser Lösung wird bei Raumtemperatur eine Lösung von 70 g S-Alaninbenzylester in 90 ml Ethanol rasch zugetropft. Es wird 2 h bei Raumtemperatur gerührt und dann die Lösung abgekühlt. Es kristallisiert das S,S-Isomere aus.

Ausbeute: 94,3 g
Fp.: 83–74°C
$[\alpha]_D^{20} = +17,8°$ (c = 1, $CH_3OH$)

Beispiel V

N-(1-S-Carbethoxy-3-oxo-3-phenyl-propyl)-S-alanin

0,5 g der Verbindung aus Beispiel IV werden in 40 ml Ethanol gelöst und 0,1 g Pd/C 10%ig zugegeben und bei Raumtemperatur und Normaldruck hydriert.

Ausbeute: 300 mg
Fp.: 210–220°C
$^1$H-NMR (DMSO-$d_6$): 1,0–1,4 (t, 6H); 3,2–5,0 (m, 8H); 7,2–8,2 (m, 5H)

**Patentansprüche für die Vertragsstaaten BE, CH, DE, FR, GB, IT, LI, LU, NL, SE**

1. Verbindung der Formel I'

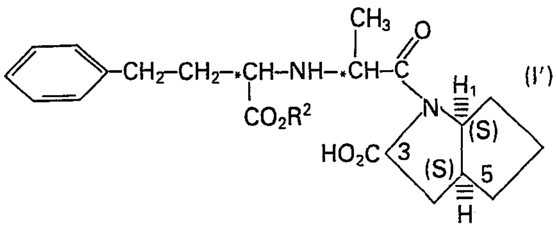

(I')

in der die Wasserstoffatome an den Brückenkopf-C-Atomen 1 und 5 zueinander cis-konfiguriert sind, die Carboxygruppen am C-Atom 3 endoständig zum bicyclischen Ringsystem orientiert (d. h. dem Cyclopentanring zugewandt) ist, und die Chiralitätszentren an den mit einem Stern (*) markierten beiden C-Atomen der Kette und am C-Atom 3 des Bicyclus in der S-Konfiguration vorliegen und in der $R^2$ = Wasserstoff, Methyl, Ethyl oder Benzyl bedeutet sowie deren physiologisch unbedenklichen Salze.

2. N-(1-S-Carbethoxy-3-phenyl-propyl)-S-alanyl-cis, endo-2-azabicyclo-[3.3.0]-octan-3-S-carbonsäure sowie deren physiologisch unbedenklichen Salze.

3. N-(1-S-Carboxy-3-phenyl-propyl)-S-alanyl-cis, endo-2-azabicyclo-[3.3.0]-octan-3-S-carbonsäure sowie deren physiologisch unbedenklichen Salze.

4. Verfahren zur Herstellung einer Verbindung gemäss einem der Ansprüche 1–3, dadurch gekennzeichnet, dass man eine Verbindung der Formel II'

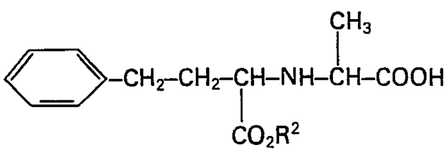

(II')

in der $R^2$ die im Anspruch 1 definierte Bedeutung mit Ausnahme von $R^2$ = Wasserstoff hat, mit einer Verbindung der Formel III

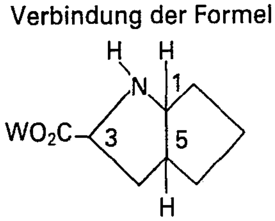

(III)

in der die Wasserstoffatome an den Brückenkopf-C-Atomen 1 und 5 zueinander cis-konfiguriert sind und die $CO_2W$-Gruppe am C-Atom 3 endoständig zum bicyclischen Ringsystem orientiert (d. h. dem Cyclopentanring zugewandt) ist und in der W eine Carboxy veresternde Gruppe bedeutet, umsetzt und anschliessend das Produkt hydriert oder mit einer Säure oder einer Base behandelt und die

erhaltene Verbindung gegebenenfalls in ihr physiologisch verträgliches Salz überführt.

5. Verfahren gemäss Anspruch 4, dadurch gekennzeichnet, dass eine Verbindung der Formel III umgesetzt wird, worin W tert. Butyl oder Benzyl bedeutet.

6. Pharmazeutisches Mittel, enthaltend eine Verbindung gemäss einem der Ansprüche 1 bis 3 und die üblichen Zusätze.

7. Verbindung gemäss einem der Ansprüche 1 bis 3 zur Anwendung als Heilmittel.

8. Verbindung gemäss einem der Ansprüche 1 bis 3 zur Anwendung als Heilmittel bei der Behandlung des Bluthochdrucks.

9. Mittel gemäss Anspruch 6, dadurch gekennzeichnet, dass es ein Diuretikum enthält.

10. Verbindung gemäss Anspruch 7 oder 8 zur Anwendung in Kombination mit einem Diuretikum.

11. Verbindung der Formel III

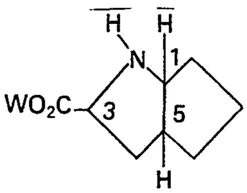

(III)

in der die Wasserstoffatome an den Brückenkopf-C-Atomen 1 und 5 zueinander cis-konfiguriert sind und die $CO_2W$-Gruppe am C-Atom 3 endoständig zum bicyclischen Ringsystem orientiert (d. h. dem Cyclopentanring zugewandt) ist und in der W Wasserstoff oder eine Carboxy veresternde Gruppe, die durch Hydrieren oder durch Behandeln mit einer Säure oder Base abspaltbar ist, bedeutet sowie deren Salze.

12. Verbindung gemäss Anspruch 11, dadurch gekennzeichnet, dass deren Chiralitätszentren alle die S-Konfiguration aufweisen.

13. Verbindung gemäss Anspruch 11 oder 12, dadurch gekennzeichnet, dass W Wasserstoff, tert. Butyl oder Benzyl bedeutet.

14. Verfahren zur Herstellung einer Verbindung der Formel III gemäss Anspruch 11, dadurch gekennzeichnet, dass man jene Verbindung, die erhalten wird, indem man eine Verbindung der Formel X

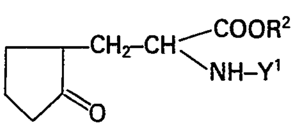

(X)

in welcher $R^2$ eine sauer abspaltbare Estergruppe und $Y^1$ einen Acylrest bedeuten, unter sauren Bedingungen cyclisiert, durch katalytische Hydrierung in Gegenwart von Edelmetallkatalysatoren oder durch Reduktion mit komplexen Hydriden in eine Verbindung der Formel III, in welcher W für Wasserstoff steht, überführt, diese gegebenenfalls zu Verbindungen der Formel III, in welcher W die Bedeutungen wie im Anspruch 11 mit der Ausnahme von Wasserstoff hat, verestert und die erhaltenen Verbindungen gegebenenfalls in ihre Salze überführt.

15. Verfahren gemäss Anspruch 14, dadurch gekennzeichnet, dass die Ausgangsverbindung des Herstellungsverfahrens für Verbindungen der Formel III erhalten wird durch Umsetzung eines Enamins der Formel VI, in welcher $X^1$ für Dialkylamino mit 2 bis 10 C-Atomen oder für einen Rest der Formel VII, worin m und o eine ganze Zahl von 1 bis 3, $(m+o) \geq 3$ und A $CH_2$, NH, O oder S bedeuten, steht,

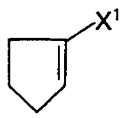

(VI)

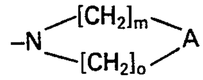

(VII)

mit einem N-acylierten β-Halogen-α-amino-carbonsäureester der Formel VIII, in welcher $X^2$ für eine nucleofuge Gruppe, $Y^1$ für Alkanoyl mit 1 bis 5 C-Atomen, Aroyl mit 7 bis 9 C-Atomen oder andere, in der Peptidchemie übliche, sauer abspaltbare Schutzgruppen und $R^2$ für Alkyl mit 1 bis 5 C-Atomen oder Aralkyl mit 7 bis 9 C-Atomen steht

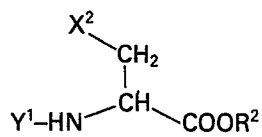

(VIII)

oder mit einem Acrylsäureester der Formel IX, in welcher $Y^1$ und $R^2$ vorstehende Bedeutung haben

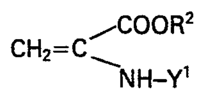

(IX)

zu einer Verbindung der Formel X, in welcher $R^2$ und $Y^1$ vorstehende Bedeutung haben

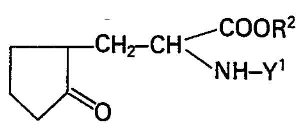

(X)

und Cyclisierung dieser Verbindung mit Hilfe starker Säuren unter Acylamid- und Esterspaltung.

**Patentansprüche für den Vertragsstaat AT**

1. Verfahren zur Herstellung einer Verbindung der Formel I'

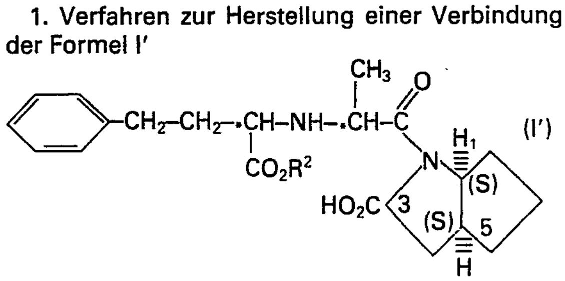

(I')

in der die Wasserstoffatome an den Brückenkopf-C-Atomen 1 und 5 zueinander cis-konfiguriert sind, die Carboxygruppen am C-Atom 3 endoständig zum bicyclischen Ringsystem orientiert (d. h. dem Cyclopentanring zugewandt) ist, und die Chiralitätszentren an den mit einem Stern (*) markierten beiden C-Atomen der Kette und am C-Atom 3 des Bicyclus in der S-Konfiguration vorliegen und in der $R^2$ = Wasserstoff, Methyl, Ethyl oder Benzyl bedeutet sowie deren physiologisch unbedenklichen Salze, dadurch gekennzeichnet, dass man eine Verbindung der Formel II'

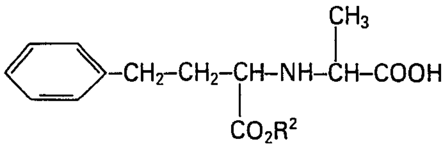

(II')

in der $R^2$ die oben definierte Bedeutung mit Ausnahme von $R^2$ = Wasserstoff hat, mit einer Verbindung der Formel III

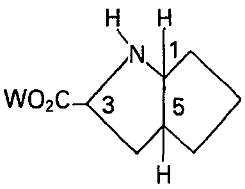

(III)

in der die Wasserstoffatome an den Brückenkopf-C-Atomen 1 und 5 zueinander cis-konfiguriert sind und die $CO_2W$-Gruppe am C-Atom 3 endoständig zum bicyclischen Ringsystem orientiert (d. h. dem Cyclopentanring zugewandt) ist und in der W eine Carboxy veresternde Gruppe bedeutet, umsetzt und anschliessend das Produkt hydriert oder mit einer Säure oder einer Base behandelt und die erhaltene Verbindung gegebenenfalls in ihr physiologisch verträgliches Salz überführt.

2. Verfahren gemäss Anspruch 1, dadurch gekennzeichnet, dass N-(1-S-Carbethoxy-3-phenyl-propyl)-S-alanyl-cis, endo-2-azabicyclo-[3.3.0]-octan-3-S-carbonsäure oder deren physiologisch verträgliches Salz hergestellt wird.

3. Verfahren gemäss Anspruch 1, dadurch gekennzeichnet, dass N-(1-S-Carboxy-3-phenyl-propyl)-S-alanyl-cis, endo-2-azabicyclo-[3.3.0]-octan-3-S-carbonsäure oder deren physiologisch verträgliches Salz hergestellt wird.

4. Verfahren gemäss einem der Ansprüche 1 bis 3, dadurch gekennzeichnet, dass eine Verbindung der Formel III umgesetzt wird, worin W tert. Butyl oder Benzyl bedeutet.

5. Verfahren zur Herstellung eines pharmazeutischen Mittels, enthaltend eine Verbindung der Formel I' gemäss einem der Ansprüche 1 bis 3, dadurch gekennzeichnet, dass man diese zusasmmen mit den üblichen Zusätzen in eine geeignete Darreichungsform bringt.

6. Verfahren gemäss Anspruch 1 zur Herstellung einer Verbindung der Formel I' zur Anwendung als Heilmittel.

7. Verfahren gemäss Anspruch 1 zur Herstellung einer Verbindung der Formel I' zur Anwendung bei der Behandlung des Bluthochdrucks.

8. Verfahren gemäss Anspruch 5, dadurch gekennzeichnet, dass man ein Diuretikum zusetzt.

9. Verfahren zur Herstellung einer Verbindung der Formel III

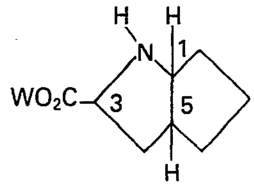

(III)

in der die Wasserstoffatome an den Brückenkopf-C-Atomen 1 und 5 zueinander cis-konfiguriert sind und die $CO_2W$-Gruppe am C-Atom 3 endoständig zum bicyclischen Ringsystem orientiert (d. h. dem Cyclopentanring zugewandt) ist und in der W Wasserstoff oder eine Carboxy veresternde Gruppe, die durch Hydrieren oder durch Behandeln mit einer Säure oder Base abspaltbar ist, bedeutet sowie deren Salze, dadurch gekennzeichnet, dass man jene Verbindung, die erhalten wird, indem man eine Verbindung der Formel X

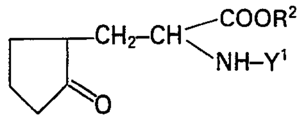

(X)

in welcher $R^2$ eine sauer abspaltbare Estergruppe und $Y^1$ einen Acylrest bedeuten, unter sauren Bedingungen cyclisiert, durch katalytische Hydrierung in Gegenwart von Edelmetallkatalysatoren oder durch Reduktion mit komplexen Hydriden in eine Verbindung der Formel III, in welcher W für Wasserstoff steht, überführt, diese gegebenenfalls zu Verbindungen der Formel III, in welcher W nicht Wasserstoff bedeutet, verestert und die erhaltenen Verbindungen gegebenenfalls in ihre Salze überführt.

10. Verfahren gemäss Anspruch 9, dadurch gekennzeichnet, dass eine Verbindung der Formel III hergestellt wird, deren Chiralitätszentren alle die S-Konfiguration aufweisen.

11.Verfahren gemäss Anspruch 9 oder 10, dadurch gekennzeichnet, dass eine Verbindung der Formel III hergestellt wird, in der W Wasserstoff, tert. Butyl oder Benzyl bedeutet.

12. Verfahren gemäss Anspruch 9, dadurch gekennzeichnet, dass die Ausgangsverbindung des Herstellungsverfahrens für Verbindungen der Formel III erhalten wird durch Umsetzung eines Enamins der Formel VI, in welcher $X^1$ für Dialkylamino mit 2 bis 10 C-Atomen oder für einen Rest der Formel VII, worin m und o eine ganze Zahl von 1 bis 3, $(m+o) \geq 3$ und A $CH_2$, NH, O oder S bedeuten, steht,

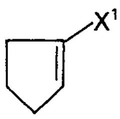

(VI)

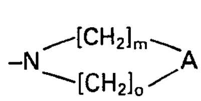

(VII)

mit einem N-acylierten β-Halogen-α-amino-carbonsäureester der Formel VIII, in welcher $X^2$ für eine nucleofuge Gruppe, $Y^1$ für Alkanoyl mit 1 bis 5 C-Atomen, Aroyl mit 7 bis 9 C-Atomen oder andere, in der Peptidchemie übliche, sauer ab-

spaltbare Schutzgruppen und $R^2$ für Alkyl mit 1 bis 5 C-Atomen oder Aralkyl mit 7 bis 9 C-Atomen steht

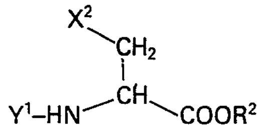

(VIII)

oder mit einem Acrylsäureester der Formel IX, in welcher $Y^1$ und $R^2$ vorstehende Bedeutung haben

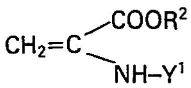

(IX)

zu einer Verbindung der Formel X, in welcher $R^2$ und $Y^1$ vorstehende Bedeutung haben

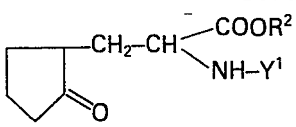

(X)

und Cyclisierung dieser Verbindung mit Hilfe starker Säuren unter Acylamid- und Esterspaltung.

**Claims for the contracting States: BE, CH, DE, FR, GB, IT, LI, LU, NL, SE**

1. A compound of the formula I'

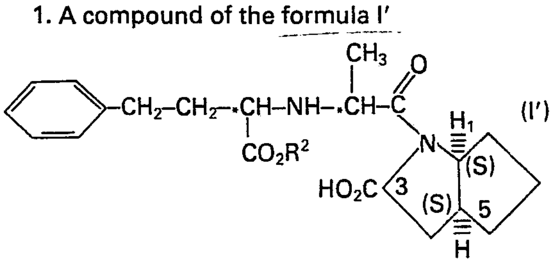

(I')

in which the hydrogen atoms on the bridge-head C atoms 1 and 5 are in the cis-configuration relative to one another and the carboxyl group on C atom 3 is orientated in the endo-position relative to the bicyclic ring system (i.e. it faces the cyclopentane ring), and the chirality centers on both C atoms labeled with a star (*) in the chain and on C atom 3 of the bicycle all have the S-configuration, and in which $R^2$ denotes hydrogen, methyl, ethy or benzyl, and physiologically acceptable salts thereof.

2. N-(1-S-Carbethoxy-3-phenyl-propyl)-S-alanyl-cis, endo-2-azabicyclo-[3.3.0]-octane-3-S-carboxylic acid and physiologically acceptable salts thereof.

3. N-(1-S-Carboxy-3-phenyl-propyl)-S-alanylcis, endo-2-azabicyclo-[3.3.0]-octane-3-S.carboxylic acid and physiologically acceptable salts thereof.

4. A process for the preparation of a compound according to any of claims 1 to 3, characterized in that a compound of the formula II'

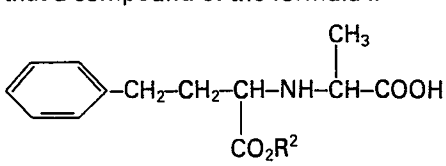

(II')

in which $R^2$ has the meaning given in claim 1, with the exception of $R^2$ = hydrogen, is reacted with a compound of the formula III

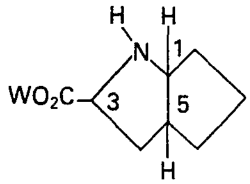

(III)

in which the hydrogen atoms on the bridge-head C atoms 1 and 5 are in the cis-configuration relative to one another and the $CO_2W$ group on C atom 3 is orientated in the endo-position relative to the bicyclic ring system (i. e. it faces the cyclopentane ring), and in which W denotes a carboxyl-esterifying group, and the product is hydrogenated or treated with an acid or a base, and the compound thus obtained is optionally converted into its physiologically acceptable salt.

5. A process according to claim 4, characterized in that a compound of the formula III, wherein W denotes tert.-butyl or benzyl, is reacted.

6. A pharmaceutical composition containing a compound according to any of claims 1 to 3 and customary additives.

7. A compound according to any of claims 1 to 3 for the use as a medicine.

8. A compound according to any of claims 1 to 3 for the use as a medicine for the treatment of hypertension.

9. A composition according to claim 6, characterized in that it contains a diuretic agent.

10. A compound according to claim 7 or 8 for the said use in combination with a diuretic agent.

11. A compound of the formula III

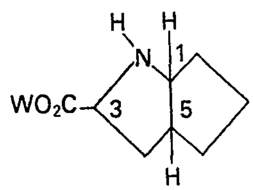

(III)

in which the hydrogen atoms on the bridge-head C atoms 1 and 5 are in the cis-configuration relative to one another and the $CO_2W$ group on C atom 3 is orientated in the endo-position relative to the bicyclic ring system (i. e. it faces the cyclopentane ring), and in which W denotes hydrogen or a carboxyl-esterifying group, which can be cleaved by hydrogenation or treatment with an acid or a base, and the salts thereof.

12. A compound according to claim 11, characterized in that its chirality centers all have the S-configuration.

13. A compound according to claim 11 or 12, characterized in that W denotes hydrogen, tert.-butyl or benzyl.

14. A process for the preparation of a compound of the formula III according to claim 11, characterized in that the compound with is obtained by cyclization of a compound of the formula X

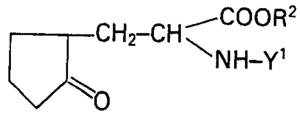

(X)

in which $R^2$ denotes an ester group which can be cleaved by an acid and $Y^1$ denotes an acyl radical, under acidic conditions, the compound obtained is converted into a compound of the formula III in which W denotes hydrogen, by catalytic hydrogenation in the presence of nobel metal catalysts or by reduction with complex hydrides, this compound is optionally esterified to give compounds of the formula III in which W has the meanings as in claim 11 with the exception of hydrogen, and the compounds obtained are optionally converted into their salts.

15. A process according to claim 14, characterized in that the starting compounds for the process for preparing compounds of the formula III are obtained by reacting an enamine of the formula VI in which $X^1$ represents dialkylamino with 2 to 10 carbon atoms or a radical of the formula VII, in which m and o denote integers from 1 to 3, $(m+o) \geq 3$ and A denotes $CH_2$, NH, O or S,

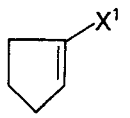

(VI)

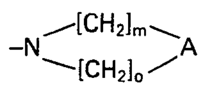

(VII)

with an N-acylated β-halogeno-α-amino-carboxylicacidester for the formula VIII, in which $X^2$ represents a nucleofugic group, $Y^1$ represents alkanoyl with 1 to 5 carbon atoms, aroyl with 7 to 9 carbon atoms or another protective group which is customary in peptide chemistry and can be split off under acid conditions, and $R^2$ represents alkyl with 1 to 5 carbon atoms or aralkyl with 7 to 9 carbon atoms,

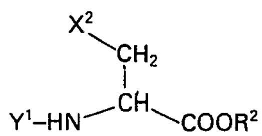

(VIII)

or with an acrylic acid ester of the formula IX, in which $Y^1$ and $R^2$ have the above meanings

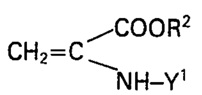

(IX)

to give a compound of the formula X, in which $R^2$ and $Y^1$ have the above meanings,

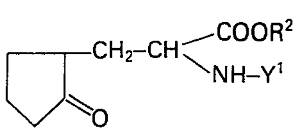

(X)

and cyclizing this compound with the aid of a strong acid, with acylamide and ester cleavage.

**Claims for the contracting State: AT**

1. A process for the preparation of a compound of the formula I'

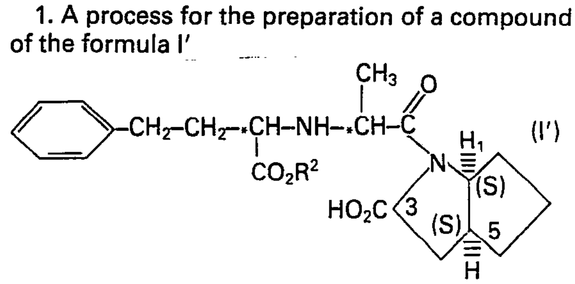

(I')

in which the hydrogen atoms on the bridge-head C atoms 1 and 5 are in the cis-configuration relative to one another and the carboxyl group on C atom 3 is orientated in the endo-position relative to the bicyclic ring system (i.e. it faces the cyclopentane ring), and the chirality centers on both C atoms labeled with a star (*) in the chain and on C atom 3 of the bicycle all have the S-configuration, and in which $R^2$ denotes hydrogen, methyl, ethy or benzyl, and physiologically acceptable salts thereof, characterized in that a compound of the formula II'

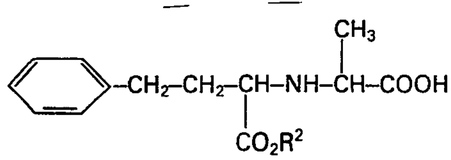

(II')

in which $R^2$ has the meaning given above, with the exception of $R^2$ = hydrogen, ist reacted with a compound of the formula III

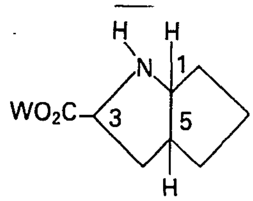

(III)

in which the hydrogen atoms on the bridge-head C atoms 1 and 5 are in the cis-configuration relative to one another and the $CO_2W$ group on C atom 3 is orientated in the endo-position relative to the bicyclic ring system (i. e. it faces the cyclopentane ring), and in which W denotes a carboxyl-esterifying group, and then the product is hydrogenated or treated with an acid or a base, and the compound thus obtained is optionally converted into its physiologically acceptable salt.

2. A process according to claim 1, characterized in that N-(1-S-carbethoxy-3-phenyl-propyl)-S-alanyl-cis, endo-2-azabicyclo-[3.3.0]-octane-3-S-carboxylic acid or a physiologically acceptable salt thereof is prepared.

3. A process according to claim 1, characterized in that N-(1-S-carboxy-3-phenyl-propyl)-S-alanyl-cis, endo-2-azabicyclo-[3.3.0]-octane-3-S-carboxylic acid or a physiologically acceptable salt thereof is prepared.

4. A process according to any of claims 1 to 3, characterized in that a compound of the formula III, wherein W denotes tert.-butyl or benzyl, is reacted.

5. A process for the preparation of a pharmaceutical composition containing a compound according to any of claims 1 to 3, characterized in that this compound together with the customary additives is brought into a suitable form for administration.

6. A process according to claim 1 for the preparation of a compound of the formula I' for the use as a medicine.

7. A process according to claim 1 for the preparation of a compound of the formula I' for the use as a medicine for the treatment of hypertension.

8. process according to claim 5 characterized in that a diuretic agent is added.

9. A process for the preparation of a compound of the formula III

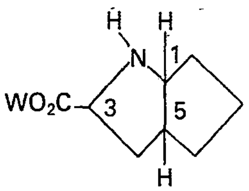

(III)

in which the hydrogen atoms on the bridge-head C atoms 1 and 5 are in the cis-configuration relative to one another and the $CO_2W$ group on C atom 3 is orientated in the endo-position relative to the bicyclic ring system (i. e. it faces the cyclopentane ring), and in which W denotes hydrogen or a carboxyl-esterifying group, which can be cleaved by hydrogenation or treatment with an acid or a base, and the salts thereof, characterized in that the compound which is obtained by cyclization of a compound of the formula X

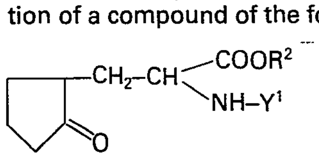

(X)

in which $R^2$ denotes an ester group which can be cleaved by an acid and $Y^1$ denotes an acyl radical, under acidic conditions, the compound obtained is converted into a compound of the formula III in which W denotes hydrogen, by catalytic hydrogenation in the presence of nobel metal catalysts or by reduction with complex hydrides, this compound is optionally esterified to give compounds of the formula III in which has the above meanings with the exception of hydrogen, and the compounds obtained are optionally converted into their salts.

10. A process according to claim 9, characterized in that a compound of the formula III is prepared wherein the chirality centers all have the S-configuration.

11. A process according to claim 9 or 10, characterized in that a compound of the formula III is prepared wherein W denotes hydrogen, tert.-butyl or benzyl.

12. A process according to claim 9, characterized in that the starting compounds for the process for preparing compounds of the formula III are obtained by reacting an enamine of the formula VI in which $X^1$ represents dialkylamino with 2 to 10 carbon atoms or a radical of the formula VII, in which m and o denote integers from 1 to 3, $(m+o) \geq 3$ and A denotes $CH_2$, NH, O or S,

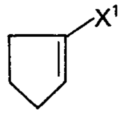

(VI)

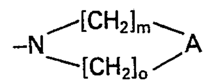

(VII)

with an N-acylated β-halogeno-α-amino-carboxylicacidester for the formula VIII, in which $X^2$ represents a nucleofugic group, $Y^1$ represents alkanoyl with 1 to 5 carbon atoms, aroyl with 7 to 9 carbon atoms or another protective group which is customary in peptide chemistry and can be split off under acid conditions, and $R^2$ represents alkyl with 1 to 5 carbon atoms or aralkyl with 7 to 9 carbon atoms,

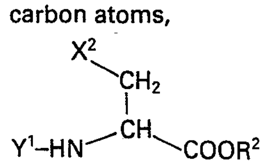

(VIII)

or with an acrylic acid ester of the formula IX, in which $Y^1$ and $R^2$ have the above meanings

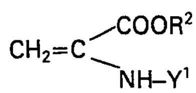

(IX)

to give a compound of the formula X, in which $R^2$ and $Y^1$ have the above meanings,

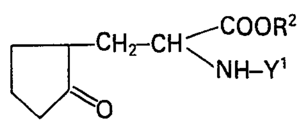

(X)

and cyclizing this compound with the aid of a strong acid, with acylamide and ester cleavage.

**Revendications pour les Etats contractants: BE, CH, DE, FR, GB, IT, LI, LU, NL, SE**

1. Composé de formule I'

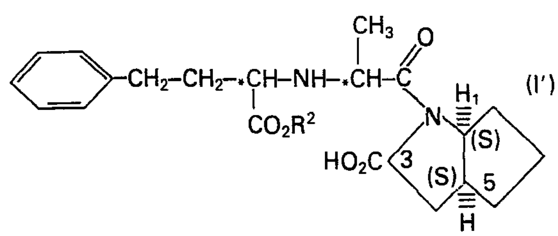

(I')

dans laquelle les atomes d'hydrogène fixés aux atomes de carbone 1 et 5 de la tête de pont ont la configuration cis l'un par rapport à l'autre, le groupe carboxylique fixé à l'atome de C en 3 est orienté en position endo par rapport au système bicyclique (c'est-à-dire qu'il est tourné vers le cycle cyclopentane), et les centres de chiralité sur les deux atomes de C de la chaîne marquée d'un astérisque (*) et sur l'atome de C en 3 du double cycle se trouvent dans la configuration S et dans laquelle $R^2$ représente l'hydrogène, un radical méthyle, éthyle ou benzyle, ainsi que leurs sels physiologiquement acceptables.

2. Acide N-(1-S-carbéthoxy-3-phényl-propyl)-S-alanyl-cis, endo-2-azabicyclo-[3.3.0]-octane-3-S-carboxylique ainsi que ses sels physiologiquement acceptables.

3. Acide N-(1-S-arboxy-3-phényl-propyl)-S-alanyl-cis, endo-2-azabicyclo-[3.3.0]-octane-3-S-carboxylique ainsi que ses sels physiologiquement acceptables.

4. Procédé de préparation d'un composé selon l'une des revendications 1 à 3, caractérisé en ce que l'on fait réagir un composé de formule II'

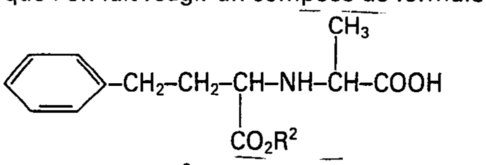

(II')

dans laquelle $R^2$ a la signification définie dans la revendication 1, à l'exception de $R^2$ = hydrogène, avec un composé de formule III

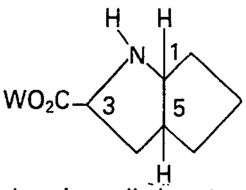

(III)

dans laquelle les atomes d'hydrogène fixés sur les atomes de carbone 1 et 5 de la tête de pont ont la configuration cis l'un par rapport à l'autre et le groupe $CO_2W$ sur l'atome de C en 3 est orienté en position endo par rapport au système bicyclique (c'est-à-dire est tourné vers le cycle cyclopentane) et dans laquelle W désigne un groupe estérifiant le groupe carboxylique et en ce qu'ensuite, on hydrogène le produit ou on le traite par un acide ou par une base et on convertit éventuellement le composé obtenu en l'un de ses sels physiologiquement acceptables.

5. Procédé selon la revendication 4, caractérisé en ce que l'on fait réagir un composé de formule III dans laquelle W désigne le radical tertiobutyle ou benzyle.

6. Médicament contenant un composé selon l'une quelconque des revendications 1 à 3 et les additifs habituels.

7. Composé selon l'une quelconque des revendications 1 à 3, destiné à l'emploi comme médicament.

8. Composé selon l'une quelconque des revendications 1 à 3, destiné à l'emploi comme médicament dans le traitement de l'hypertension.

9. Médicament selon la revendication 6, caractérisé en ce qu'il contient un diurétique.

10. Composé selon la revendication 7 ou 8, destiné à l'emploi en association avec un diurétique.

11. Composé de formule III

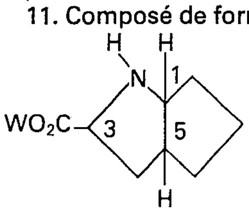

(III)

dans laquelle les atomes d'hydrogène fixés sur les atomes de carbone 1 et 5 de la tête de pont ont la configuration cis l'un par rapport à l'autre et le groupe $CO_2W$ fixé sur l'atome de C en 3 est orienté en position endo par rapport au système bicyclique (c'est-à-dire tourné vers le cycle cyclopentane) et dans laquelle W représente l'hydrogène ou un groupe estérifiant le groupe carboxylique, qui peut être séparé par hydrogénation ou par traitement avec un acide ou avec une base ainsi que ses sels.

12. Composé selon la revendication 11, caractérisé en ce que ses centres de chiralité présentent tous la configuration S.

13. Composé selon la revendication 11 ou 12, caractérisé en ce que W représente l'hydrogène, le radical tertiobutyle ou le radical benzyle.

14. Procédé de préparation d'un composé de formule III selon la revendication 11, caractérisé en ce que l'on part du composé obtenu par cyclisation dans des conditions acides d'un composé de formule X

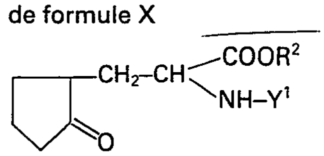

(X)

dans laquelle $R^2$ représente un groupe ester séparable par action d'un acide et $Y^1$ représente un radical acyle, que l'on convertit le composé ainsi obtenu, par hydrogénation catalytique, en présence de catalyseurs à base de métaux nobles ou par réduction par des hydrures complexes, en un composé de formule III, dans laquelle W représente l'hydrogène, que l'on estérifie éventuellement celui-ci pour donner des composés de formule III dans laquelle W a les significations indiquées dans la revendication 11 à l'exception de l'hydrogène, et que l'on convertit éventuellement les composés obtenus en leurs sels.

15. Procédé selon la revendication 14, caractérisé en ce que l'on obtient le composé de départ du procédé de préparation des composés de formule III en faisant réagir une énamine de formule VI, dans laquelle $X^1$ représente un groupe dialkylamino ayant de 2 à 10 atomes de carbone ou un radical de formule VII dans laquelle m et o sont des nombres entiers de 1 à 3, $(m+o) \geq 3$ et A représente $CH_2$, NH, O ou S,

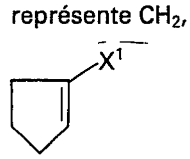

(VI)

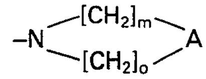

(VII)

avec un ester N-acylé d'un acide β-halogéno-α-amino-carboxylique de formule VIII, dans laquelle $X^2$ représente un groupe nucléofuge, $Y^1$ représente un groupe alcanoyle ayant 1 à 5 atomes de C, un groupe aroyle ayant 7 à 9 atomes de C ou d'autres groupes protecteurs séparables par action d'un acide et usuels dans la chimie des peptides et $R^2$ représente un groupe alkyle ayant 1 à 5

atomes de carbone ou un groupe aralkyle ayant 7 à 9 atomes de carbone

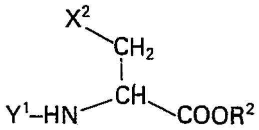

(VIII)

ou avec un ester d'acide acrylique de formule IX, dans laquelle $Y^1$ et $R^2$ ont la signification ci-dessus

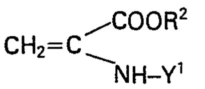

(IX)

pour donner un composé de formule X, dans laquelle $R^2$ et $Y^1$ ont la signification ci-dessus,

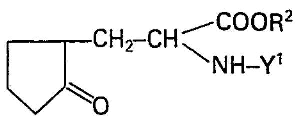

(X)

et en cyclisant ce composé à l'aide d'acides forts avec séparation des groupes acylamide et ester.

**Revendications pour l'Etats contractant: AT**

1. Procédé de préparation d'un composé de formule I'

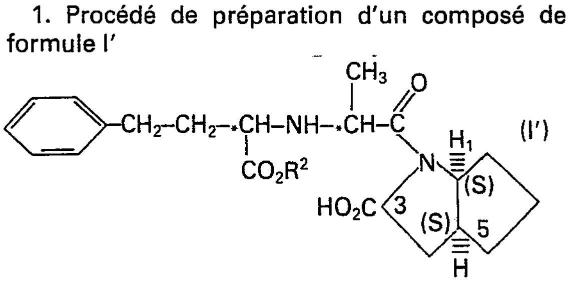

(I')

dans laquelle les atomes d'hydrogène fixés sur les atomes de carbone 1 et 5 de la tête de pont ont une configuration cis l'un par rapport à l'autre, le groupe carboxylique fixé à l'atome de C en 3 est orienté en position endo par rapport au système bicyclique (c'est-à-dire tourné vers le cycle cyclopentane), et les centres de chiralité sur les deux atomes de C de la chaîne marqués d'un astérisque (*) et sur l'atome de C en 3 du double cycle ont la configuration S et dans laquelle $R^2$ représente l'hydrogène, un radical méthyle, éthyle ou benzyle, ainsi que leurs sels physiologiquement acceptables, caractérisé en ce que l'on fait réagir un composé de formule II'

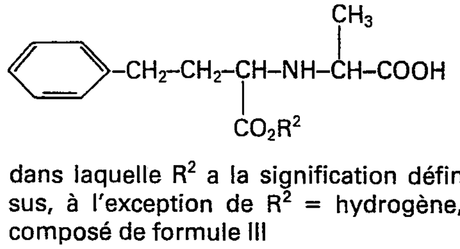

(II')

dans laquelle $R^2$ a la signification définie ci-dessus, à l'exception de $R^2$ = hydrogène, avec un composé de formule III

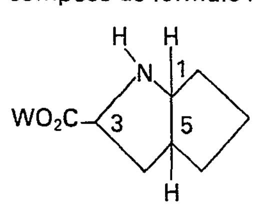

(III)

dans laquelle les atomes d'hydrogène fixés sur les atomes de carbone 1 et 5 de la tête de pont ont la configuration cis l'un par rapport à l'autre et le groupe $CO_2W$ fixé sur l'atome de C en 3 est orienté en position endo par rapport au système bicyclique (c'est-à-dire tourné vers le cycle cyclopentane) et dans laquelle W représente un groupe estérifiant le groupe carboxylique, et en ce qu'ensuite, on hydrogène le produit ou on le traite par un acide ou par une base et on convertit éventuellement le composé obtenu en l'un de ses sels physiologiquement acceptables.

2. Procédé selon la revendication 1, caractérisé en ce que l'on prépare l'acide N-(1-S-carbéthoxy-3-phényl-propyl)-S-alanyl-cis, endo-2-azabicyclo-[3.3.0]-octane-3-S-carboxylique ou un de ses sels physiologiquement acceptables.

3. Procédé selon la revendication 1, caractérisé en ce que l'on prépare l'acide N-(1-S-carboxy-3-phényl-propyl)-S-alanyl-cis, endo-2-azabicyclo-[3.3.0]-octane-3-S-carboxylique ou un de ses sels physiologiquement acceptables.

4. Procédé selon l'une quelconque des revendications 1 à 3, caractérisé en ce que l'on fait réagir un composé dè formule III dans laquelle W représente le radical tertiobutyle ou benzyle.

5. Procédé de préparation d'un médicament contenant un composé de formule I' selon l'une des revendications 1 à 3, caractérisé en ce que l'on met celui-ci, avec les additifs habituels, sous une forme d'administration appropriée.

6. Procédé selon la revendication 1 pour la préparation d'un composé de formule I' destiné à l'emploi comme médicament.

7. Procédé selon la revendication 1 pour la préparation d'un composé de formule I' destiné au traitement de l'hypertension.

8. Procédé selon la revendication 5, caractérisé en ce que l'on ajoute un diurétique.

9. Procédé de préparation d'un composé de formule III

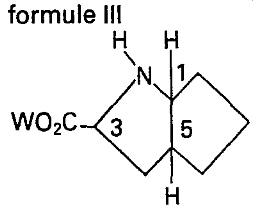

(III)

dans laquelle les atomes d'hydrogène fixés sur les atomes de carbone 1 et 5 de la tête de pont ont la configuration cis l'un par rapport à l'autre et le groupe $CO_2W$ fixé sur l'atome de C en 3 est orienté en position endo par rapport au système bicyclique (c'est-à-dire tourné vers le cycle cyclopentane) et dans laquelle W représente l'hydrogène ou un groupe estérifiant le groupe carboxylique, qui peut être séparé par hydrogénation ou par traitement avec un acide ou une base, ainsi que les sels de ce composé, caractérisé en ce que l'on part du composé que l'on obtient en cyclisant dans des conditions acides un composé de formule X

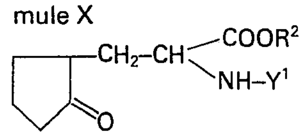

(X)

dans laquelle $R^2$ représente un groupe ester séparable par action d'un acide et $Y^1$ représente un radical acyle, que l'on convertit le composé ainsi obtenu, par hydrogénation catalytique, en présence de catalyseurs à base de métaux nobles ou par réduction par des hydrures complexes, en un composé de formule III, dans laquelle W représente l'hydrogène, que l'on estérifie éventuellement celui-ci pour donner des composés de formule III dans laquelle W est défini comme ci-dessus, mais ne représente par l'hydrogéne, et en ce que l'on convertit éventuellement les composés obtenus en leurs sels.

10. Procédé selon la revendication 9, caractérisé en ce que l'on prépare un composé de formule III dont les centres de chiralité présentent tous la configuration S.

11. Procédé selon la revendication 9 ou 10, caractérisé en ce que l'on prépare un composé de formule III dans laquelle W représente l'hydrogène, un radical tertiobutyle ou un radical benzyle.

12. Procédé selon la revendication 9, caractérisé en ce que le composé de départ du procédé de fabrication des composés de formule III est obtenu par réaction d'une énamine de formule VI, dans laquelle $X^1$ représente un groupe dialkylamino ayant de 2 à 10 atomes de C ou un radical de formule VII dans lequel m et o sont des nombres entiers de 1 à 3, (m+o) ≥3 et A représente $CH_2$, NH, O ou S,

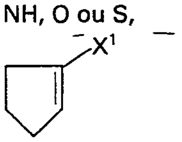

(VI)

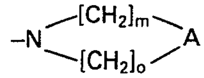

(VII)

avec un ester N-acylé d'un acide β-halogéno-α-amino-carboxylique de formule VIII, dans laquelle $X^2$ représente un groupe nucléofuge, $Y^1$ représente un groupe alcanoyle ayant 1 à 5 atomes de C, un groupe aroyle ayant 7 à 9 atomes de C ou d'autres groupes protecteurs séparables par action d'un acide et usuels dans la chimie des peptides et $R^2$ représente un groupe alkyle ayant 1 à 5 atomes de carbone ou un groupe aralkyle ayant 7 à 9 atomes de carbone

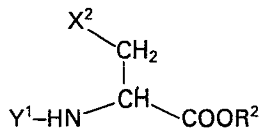

(VIII)

ou avec un ester d'acide acrylique de formule IX, dans laquelle $Y^1$ et $R^2$ ont la signification ci-dessus

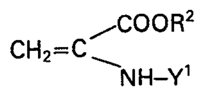

(IX)

pour donner un composé de formule X, dans laquelle $R^2$ et $Y^1$ ont la signification ci-dessus,

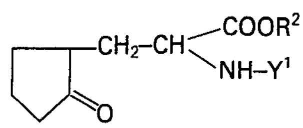

(X)

et en cyclisant ce composé à l'aide d'acides forts avec séparation des groupes acylamide et ester.